# EUROPEAN PATENT APPLICATION

(11) **EP 0 748 784 A1**
(43) Date of publication of application: **18.12.1996**
(21) Application number: 96108661.8
(22) Date of filing: 30.05.1996
(51) Int. Cl.: C07C 2/62, B01J 31/02, B01J 31/40

(54) **Process for the preparation of alkylated aromatic compounds**

(30) Priority: 13.06.1995 DK 672/95
(71) Applicant: Haldor Topsoe A/S, DK-2800 Lyngby (DK)
(72) Inventor: Hommeltoft, Sven Ivar, 3400 Hillerod (DK)

(57) **Abstract**

Process for liquid phase alkylation of aromatic hydrocarbons with an olefinic alkylating agent, comprising the steps of passing a process stream containing aromatic hydrocarbons and the alkylating agent at alkylating conditions through a fixed bed alkylation reactor of particulate polar contact material in the presence of a fluorinated sulphonic acid catalyst, and withdrawing a product stream of alkylated product.

## Description

The present invention relates to the preparation of aromatic hydrocarbons, and in particular to liquid phase alkylation of aromatic hydrocarbons in the presence of a supported liquid acid catalyst.

Alkylation of aromatic hydrocarbons is conventionally performed by Friedel-Craft's synthesis employing a Lewis acid, e.g. AlCl₃ and BF₃ in the reaction of aliphatic hydrocarbon halides or olefinic hydrocarbons with aromatic compounds. The reaction is carried out in liquid phase by mixing the reactants in the presence of a Lewis acid and stirring the mixture until the alkylation reaction is completed.

In the past, certain improvements in the alkylation process of aromatic hydrocarbons have been suggested, including alkylation of benzene with alcohols in presence of sulphuric acid catalyst (Bull Natl. Res. Cent. (Egypt) 1993, 18(1)) and use of zeolitic materials as catalysts in the alkylation of aromatic compounds (WO Patent Publication No. 93/00992).

It has now been found that aromatic hydrocarbon compounds may be alkylated with high selectivity by reaction with olefins in a fixed bed alkylation reactor having adsorbed on a particulate contact material a fluorinated sulphonic acid catalyst.

Pursuant to this finding, the present invention provides an improved process for liquid phase alkylation of aromatic hydrocarbons with an olefinic alkylating agent, comprising steps of passing a process stream containing aromatic hydrocarbons and the alkylating agent at alkylating conditions through a fixed bed alkylation reactor of particulate polar contact material in the presence of a fluorinated sulphonic acid catalyst, and withdrawing a product stream of alkylated aromatic hydrocarbon product.

Based on different migration velocities of the less polar hydrocarbon process stream having a low polarity and the high polar fluorinated sulphonic acid catalyst on a polar contact material, a preferred embodiment of the invention comprises the further process steps of
establishing within a confined area of the polar contact material a movable reaction zone with the fluorinated sulphonic acid catalyst adsorbed on the contact material;
passing a process stream of the aromatic hydrocarbon and the olefinic alkylating agent at alkylating conditions in one flow direction through the reaction zone and the residue of the polar contact material; and optionally
periodically reversing the flow direction of the process stream as the reaction zone has passed substantially the whole length of the contact material.

Preferred fluorinated sulphonic acid catalysts for use in the inventive process are the C₁-C₅ perfluoro-alkylsulphonic acids. Presently, the most preferred acid catalyst is trifluoromethanesulphonic acid.

Compared to the known alkylation catalysts, the acidity of the fluorinated sulphonic acid catalyst is considerably higher resulting in improved efficiency in the alkylation of aromatic hydrocarbons.

Due to high efficiency and stability of the fluorinated sulphonic acid catalyst during alkylation reactions, small amounts of the acid applied on the contact material ensure high yields of alkylated products. Thus for instance, when using silica as contact material, amounts of between 0.1 and 200 ml, preferably 1-100 ml, and most preferred 5-50 ml acid per cm² cross-sectional area occupied by the contact material in the reaction zone, will be sufficient to provide high alkylation rates.

Beside the above silica contact material, any of the polar and non-basic refractory materials are suitable for use as contact materials in the inventive process. Preferred materials are silica, aluminum borate, boron phosphate, boron sulphate, zirconia, titania, niobium oxides, tin oxides or mixtures thereof.

By interaction of polar groups of the contact material molecules with polar groups of the fluorinated sulphonic acid catalyst molecule, the acid is adsorbed strongly on the contact material within a confined area, thereby providing the reaction zone for the alkylation reaction. The band width of adsorbed acid, wherein the alkylation reaction proceeds, is determined by the actual contact material used.

Within the reaction zone, a process stream of aromatic hydrocarbons, such as benzene and olefinic alkylating agent, typically C₂-C₂₀ olefins are converted at alkylating conditions to a product stream containing alkylated aromatic hydrocarbon product by catalysis of the fluorinated sulphonic acid adsorbed on the contact material. The process stream may be passed through the alkylation reactor at temperatures of between -40° and 100°C and at a pressure varying in the range of between 1-100 bar abs. depending on the composition of the process stream and the actual reaction temperature.

The weight ratio of aromatic substrate to alkylating agent in the process stream may, thereby, vary between 1.5:1 and 30:1.

During the alkylation reaction, the acid catalyst and, consequently, the reaction zone moves continuously to a new position located nearer the outlet end of the alkylation reactor by interaction with the process stream flowing through and reacting in the zone.

As a theoretical explanation, the elution of the fluorinated sulphonic acid catalyst is caused by reactions of catalyst with olefins in the process stream to a fluorinated sulphonic acid ester, which is less polar than the original acid and more loosely adsorbed on the contact material in the reaction zone. The ester moves together with the process stream until it is cleaved to the free acid and a carbonium-ion, which reacts with the aromatic compound to form an alkylaromatic compound.

The migration velocity of the acid catalyst in the reactor and on the contact material is as mentioned herein before much lower than the migration velocity of the hydrocarbons in the process and product stream resulting in a much longer elution time for the acid catalyst than the hydrocarbon process stream.

During the migration of the acid catalyst and the reaction zone on the contact material, the catalytic activity of the fluorinated sulphonic acid is substantially retained and the acid is still catalytic active, when the reaction zone reaches the reactor outlet.

It is thus possible to reuse the acid as it reaches the outlet end of the alkylation reactor in a subsequent process cycle without recovery of the acid. Thereby, the flow direction of the process stream introduced into the alkylation reactor is reversed, and the reaction zone pushed towards the opposite end of the reactor by interaction with the process stream as described above.

By periodically reversing the flow direction of the process stream and pushing the reaction zone inside the reactor between the opposite ends of the contact material bed even very small amounts of acid catalyst ensure high yields of alkylated aromatic products without restitution or recovery of spent catalyst for a considerable time on stream.

When reversing the flow direction of the process stream a small volume of the stream introduced just before the flow reversion will not have passed through the reaction zone, and, consequently, leave the reactor unconverted.

Thus, in a further embodiment of the invention the volume of the process stream, which is withdrawn from the alkylation reactor during the reversion of the flow direction, is recycled to the inlet of the alkylation reactor.

Alternatively and instead of recycling a part of the process stream during the flow reversion, the stream leaving the alkylation reactor at the beginning of each process cycle may be conducted to a subsequent alkylation reactor and processed in similar manner as in the previous reactor.

When carrying out the process in at least two reactors connected in series, a process stream of aromatic hydrocarbon substrate and alkylating agent is sequentially passed in one direction through the reactors being alternatingly provided with a reaction zone of the fluorinated sulphonic acid catalyst adsorbed within a confined area of a polar contact material arranged in the reactors. The acid catalyst is thereby cycled with the process stream sequentially through the first and second reactor and recycled back to the first reactor, whenever it leaves the second reactor without reversing the flow direction of the process stream. Because the acid catalyst is cycled between at least two reactors, an attendant advantage of this embodiment is that the contact material in the reactors can be flushed after the acid catalyst has been transferred to the following reactor in the series. Thereby, residual unconverted alkylation reactants in the reactor are brought in contact with the acid catalyst in the reactor containing the acid catalyst.

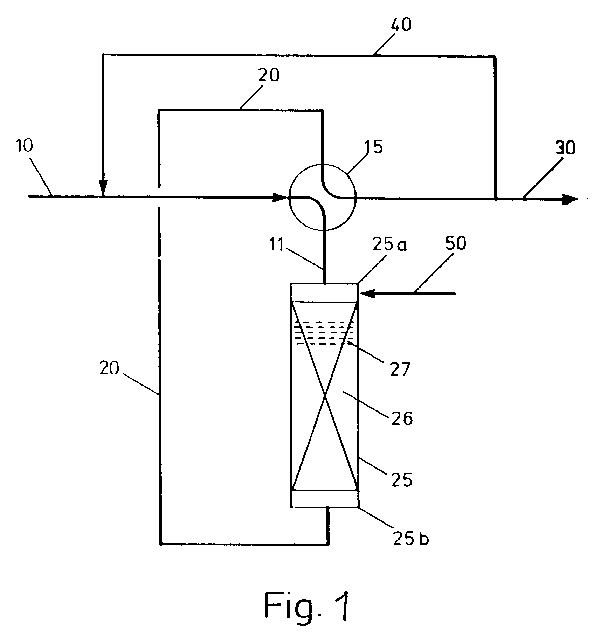

Certain aspects and features of the invention are further illustrated by reference to the drawing, in which
Fig. 1 is a process diagram of an alkylation process, wherein the fluorinated sulphonic acid catalyst is arranged as a moveable reaction zone on particulate contact material according to a preferred embodiment of the invention.

A process stream of aromatic hydrocarbon substrate and olefinic alkylating agent is passed in line 10 to four-way valve 15 for further distribution of the stream to alkylation reactor 25, controlled by the actual adjustment of the valve. The alkylation reactor 25 is loaded with a fixed bed of particulate polar contact material 26, whereon a reaction zone 27 is established by applying fluorinated sulphonic acid introduced through line 50.

As indicated in the Figure, the reaction zone 27 is in an initial cycle of the alkylation process located near end 25a of reactor 26 and the process stream is appropriately introduced via line 11 into reactor 25 at end 25a by adjustment of valve 15 to give passage from line 10 to line 11.

Thereby, the process stream is forced to pass from end 25a through reaction zone 27 to end 25b of reactor 25. In reaction zone 27 the hydrocarbon substrate reacts with the olefinic alkylating agent catalyzed by the fluorinated sulphonic acid adsorbed on contact material 26. A product stream containing alkylated products is withdrawn through line 20 at end 25b of reactor 25. At the shown adjustment of valve 15, line 20 is connected to product lines 21 and 30 through which the product stream is passed to a storage tank (not shown) or to further processing.

During the initial cycle of the process, the reaction zone 27 moves from end 25a to end 25b of reactor 25 by interaction with aromatic hydrocarbons in the process stream as described above. After having reached a position near end 25b the flow direction of the process stream in reactor 25 is in a subsequent cycle of the alkylation process reversed by adjustment of valve 15 to give passage from line 10 to line 20. At this valve adjustment, the process stream in line 20 is introduced at end 25b into reactor 25 and reaction zone 27 is pushed towards end 25a of the reactor. Thereby, the product stream is withdrawn at end 25a of reactor 25 through line 11, which is connected to line 21 via valve 15.

At the beginning of each process cycle the volume of the process stream, which is introduced into the reactor 25 before the reversal of flow direction immediately and has not yet passed through reaction zone 27, is withdrawn unreacted from reactor 25. The unreacted volume is in the initial phase of each process cycle recycled from line 21 via recycle line 40 to feed line 10.

### Example 1-6

The Examples were performed by using a stainless steel reactor tube with an outer diameter of 1/4" and a length of 6 m. The reactor tube was packed with 100 ml silica gel (Merck 100, 0.2-0.5 mm particle size) contact material. 6 ml of trifluoromethanesulphonic acid catalyst were applied on the contact material at the inlet end of the reactor tube. During alkylation, the reactor temperature was maintained at 40°C by submerging the tube in a thermostated water bath.

### Example 1

A reaction mixture of 3%(w/w) ethylene alkylation agent in benzene substrate was passed at a feed rate of 2.5/min. through the above reactor tube resulting in an alkylated product consisting of 8% (w/w) ethyl benzene, 0.4% (w/w) diethyl benzene, less than 0.03% (w/w) triethyl benzene and benzene as balance.

### Example 2

Benzene was alkylated with propene by a similar procedure to that of Example 1. A reaction mixture of 6%(w/w) propene in benzene results in a product of 13% (w/w) isopropyl benzene, 2%(w/w) diisopropyl benzene 0.2%(w/w) triisopropyl benzene and benzene as balance.

### Example 3

Similar to the above Examples, a reaction mixture of 5%(w/w) 2-butene in benzene was alkylated resulting in a product of 10%(w/w) monobutyl benzene, 1%(w/w) dibutyl benzene and less than 0.1%(w/w) tributyl benzene with benzene as balance.

### Example 4

Simultaneously, alkylation of benzene and isobutane with propene alkylating agent.

A mixture of 10%(w/w) benzene, 5%(w/w) propene in 85%(w/w) isobutane was alkylated in the above described reactor.

A product stream of alkylated aliphatic and aromatic compounds was withdrawn. The aromatic product fraction consisted of 34%(w/w) benzene, 27%(w/w) monoalkyl benzene, 20%(w/w) dialkyl benzenes and 19%(w/w) trialkyl benzene.

### Example 5

In an alkylation process similar to that of Example 4, a reaction mixture of 20%(w/w) benzene, 4%(w/w) 2-butene in 76%(w/w) isobutene, a product of alkylated aliphatic and aromatic hydrocarbon was obtained. The composition of the aromatic fraction contained 69%(w/w) benzene, 23%(w/w) butyl benzene, 7%(w/w) dibutyl benzene and 1%(w/w) tributyl benzene.

### Example 6

A reaction mixture of 10%(w/w) benzene, 5%(w/w) propene and 5%(w/w) 1-butene alkylating agent in 80%(w/w) isobutane was correspondingly alkylated at a procedure similar to that of Example 4. A product of alkylated aliphatic and aromatic compounds was withdrawn. The composition of the aromatic product fraction was : 17%(w/w) benzene, 12%(w/w) isopropyl benzene, 12%(w/w) butyl benzene, 31%(w/w) dialkyl benzenes and 28%(w/w) trialkyl benzenes with 1.8% (w/w) tributyl benzene.

## Claims

1. Process for liquid phase alkylation of aromatic hydrocarbons with an olefinic alkylating agent, comprising the steps of passing a process stream containing aromatic hydrocarbons and the alkylating agent at alkylating conditions through a fixed bed alkylation reactor of particulate polar contact material in the presence of a fluorinated sulphonic acid catalyst, and withdrawing a product stream of alkylated product.

2. The process of claim 1, comprising the further steps of establishing within a confined area of the polar contact material a movable reaction zone with the fluorinated sulphonic acid catalyst adsorbed on the contact material;
passing the process stream of the aromatic hydrocarbon and the olefinic alkylating agent at alkylating conditions in one flow direction through the reaction zone and the residue of the polar contact material; and optionally
periodically reversing the flow direction of the process stream as the reaction zone has passed substantially the whole length of the contact material.

3. The process of claim 1, wherein the fluorinated sulphonic acid catalyst is selected from C₁-C₅ perfluoroalkyl sulphonic acids.

4. The process of claim 1, wherein the fluorinated sulphonic acid catalyst consists of trifluoromethanesulphonic acid.

5. The process of claim 1, wherein the polar contact material is selected from silica, aluminum borate, boron phosphate, boron sulphate, alumina, titania, zirconia, niobium oxides, tin oxides and mixtures thereof.

6. The process of claim 1, wherein the polar contact material consists of silica.

7. The process of claim 1, wherein the alkylating agent is selected from C₂-C₂₀ olefinic hydrocarbons.

8. Process according to any one of the preceding claims, wherein the process stream further comprises a mixture of aromatic and aliphatic hydrocarbons and the alkylated product comprises alkylated aromatic and alkylated aliphatic hydrocarbons.
